# EUROPEAN PATENT APPLICATION

(11) **EP 1 447 087 A1**
(43) Date of publication of application: **18.08.2004**
(21) Application number: 02802059.2
(22) Date of filing: 24.10.2002
(51) Int. Cl.: A61K 33/24, A61P 43/00

(54) **SUPERFINE AQUEOUS DISPERSION OF NOBLE METAL FOR HEAD MASSAGE**

(30) Priority: 25.10.2001 JP 2001327410
(71) Applicant: Phild Co., Ltd., Kyoto city, Kyoto pref. 604-8152 (JP)
(72) Inventor: HIRATA, Yoshihiro, c/o Phild co.,LTD, Nakagyo-ku, Kyoto city 604-8152 (JP)
(74) Representative: VOSSIUS & PARTNER
(86) International application number: PCT/JP2002/011027
(87) International publication number: WO 2003/035084

(57) **Abstract**

When micro-dispersion water containing super-fine noble metal particles, as produced by causing plasma discharge in water between a noble metal electrode and its counter electrode, is applied to the scalp and the head is massaged, such solution will permeate through the hair roots into the hair tissues in the scalp all the way to the hair matrix cells and hair papilla, thereby transferring the beneficial effects of such solution to the tissue nerves and capillary vessels to activate the brains.

## Description

### Field of the Invention

This invention relates to a head massaging solution in which super-fine noble metal particles are micro-dispersed, wherein the solution can be suitably applied to the scalp for massaging of the head.

### Background of the Invention

Noble metals such as gold, platinum and silver have been considered some of the most valuable metals available to men since the ancient times and used chiefly in ornaments and treasures. The efficacies of these metals in the promotion of human health have also been recognized of late, and noble metals are now used in such applications as health bracelets made of pure gold or Japanese sake containing gold foil. In reality, however, these "health" products failed to prove sufficient efficacies on human health beyond their luxury image derived from the use of expensive noble metals.

In recent years, however, the health functions of noble metals have been again drawing the attention of the increasingly health-conscious public. In particular, noble metals in ion or fine particle form are now known to offer more remarkable health-promoting function than noble metals themselves or in any processed form for the purpose of decoration, such as gold foil.

The potential health-promoting function and disease-curing effect of noble metal ions and fine noble metal particles are generating strong interests. The significant contributions of noble metals to health are also known. In applying noble metals, use of aqueous solution or water dispersion of noble metal ions and fine noble metal particles presents a significant potential. However, there are several problems that must be resolved before these applications can be put to commercial use, and therefore further technological developments in this field have been eagerly awaited.

For example, dissolving noble metal ions or fine noble metal particles in water is very difficult. For this reason, the conventional methods required high production costs and therefore did not provide desired economy. They also posed other problems, such as insufficient utility of the obtained products in promoting health.

On the other hand, various methods for massaging the scalp have been reported to date (Japanese Patent Application Laid-open No. 7-16304), and equipment used for scalp massaging purposes have also been developed (Japanese Patent Application Laid-open Nos. 8-688, 11-47211 and 11-196925). However, all these methods and equipment were aimed to promote hair growth. Specifically, they were designed to promote hair growth by cleaning the scalp, promoting blood circulation under the scalp via application of a hair tonic or heat, or using head massage in conjunction.

### Disclosure of the Invention

The inventors developed new methods for micro-dispersing super-fine noble metal particles in water, including one wherein a mixture gas of oxygen and hydrogen is burned in high-pressure water in which flakes of noble metal foil are floating and then the combustion gas is used to heat/melt the noble metal foil, and the other wherein plasma discharge is caused in water between a noble metal electrode and its counter electrode and then the generated noble metal ion vapor is caused to contact, and micro-disperse in, water. In light of the fact that these methods have made it possible to obtain micro-dispersion water of super-fine noble metal particles, the inventors have applied the obtained micro-dispersion water of super-fine noble metal particles in a manner embodying the health-promotion function and disease-curing effect of noble metals. That is, the inventors have successfully produced micro-dispersion water of super-fine noble metal particles, which, if applied to the scalp and followed by head massage, effectively activates the brains.

Specifically, the present invention is a successful development of micro-dispersion water of super-fine noble metal particles, wherein the micro-dispersion water utilizes the excellent electrical stability of super-fine noble metal particles to facilitate the transmissions of electrical signals through the nerves, improve the functions of our brains and nerves in controlling the various parts of our body, correct the autonomic nerves functions, stabilize the hormonal balance and improve the motor and sensory functions, if the micro-dispersion water of super-fine noble metal particles is applied to the head and then the head is massaged.

The present invention provides a desired head massaging solution comprising a micro-dispersion solution of super-fine noble metal particles, by means of causing plasma discharge in water between a noble metal electrode and its counter electrode.

The present invention allows for activation of the brains through a head massaging care utilizing super-fine noble metal particles, which comprises applying the aforementioned micro-dispersion solution of super-fine noble metal particles to the head, cleaning the scalp by shampooing, and drying.

The basic application modes of noble metal materials include aqueous solution or water dispersion of ions or fine particles. However, there are several problems that must be resolved before these applications can be put to commercial use. For example, dissolving noble metal ions or fine noble metal particles in water is very difficult, and the only methods known to date are limited to those that mix gold foil or powder in water or produce gold electrolyte. However, these conventional methods required high production costs and therefore did not provide desired economy. In addition, they did not prove sufficient effectiveness in the promotion of health.

The inventors diligently studied ways to improve these conventional technologies and ultimately produce micro-dispersion water of super-fine noble metal particles. In other words, the present invention is based on the assumption that micro-dispersing super-fine noble metal particles of micron to nano-scale that are smaller than fine noble metal particles would improve the various drawbacks mentioned above. After trial and error, the inventors developed a technology to cause plasma discharge in water between a noble metal electrode and its counter electrode, both equipped on a high-voltage discharge generator, and then cause the generated noble metal ion vapor to contact, and micro-disperse in, water, thereby producing water in which super-fine noble metal particles are micro-dispersed. This technology has led to the present invention.

One feature of the present invention is that it has realized a production and utilization of water in which super-fine noble metal particles of micron to nano-order are micro-dispersed, which are finer than the normal fine noble metal particles as mentioned above. This way, stable micro-dispersion water in which super-fine noble metal particles remain dispersed, without settling, for a longer period of time compared with water in which flakes of noble metal foil or fine noble metal particles are simply floating. Its physical properties make the obtained water safe for human consumption just like normal water, and the water also provides high functions in promoting health, among others.

### Brief Explanation of the Drawings

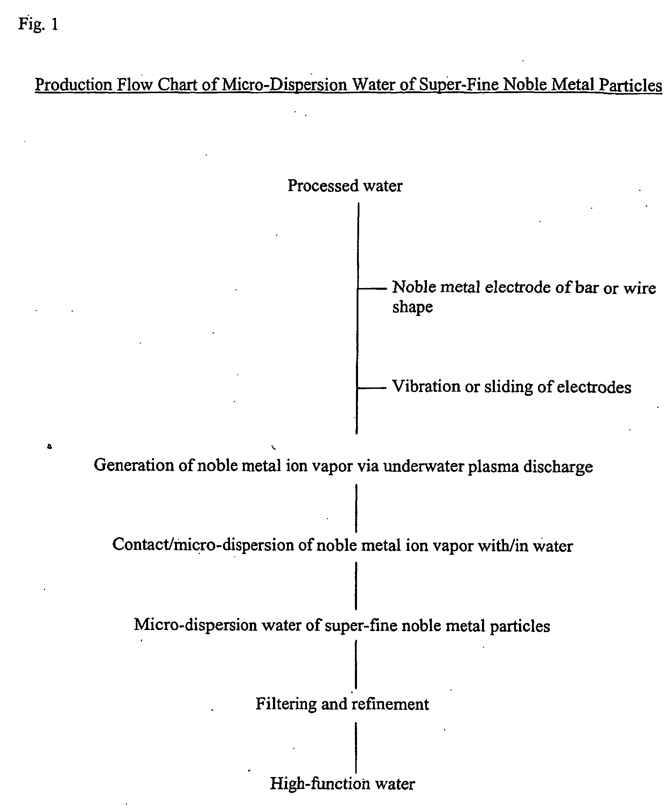

### "Fig. 1"

A production flowchart of micro-dispersion water of super-fine noble metal particles as proposed by the present invention

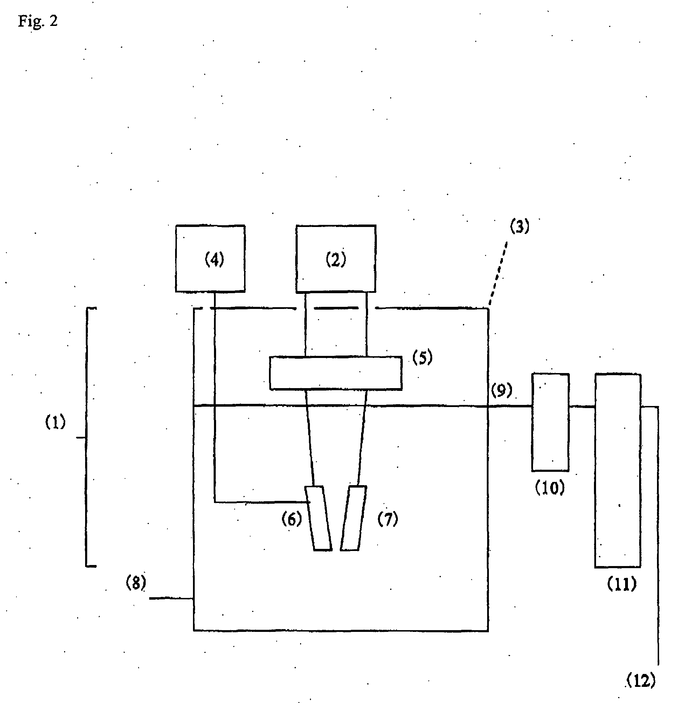

### "Fig. 2"

An apparatus for producing the aforementioned micro-dispersion water of super-fine noble metal particles

### Description of the Symbols

- 1:: High-voltage discharge generator
- 2:: Power supply for high-voltage/current discharge
- 3:: Water tank
- 4:: Electrode feeder
- 5:: Electrode-vibrating/sliding device
- 6:: Noble metal electrode
- 7:: Counter electrode
- 8:: Water inlet to water tank
- 9:: Outlet for micro-dispersion water of super-fine noble metal particles
- 10:: Discharge pump for micro-dispersion water of super-fine noble metal particles
- 11:: Filter system
- 12:: Product

### Best Mode for Carrying Out the Invention

The present invention provides a head massaging solution comprising micro-dispersion water in which super-fine noble metal particles are micro-dispersed, as produced by the aforementioned method, wherein application of the head massaging solution to the head followed by head massage will activate the brains.

Fine noble metal particles do not dissolve in water, and therefore even when a dispersed state of fine noble metal particles is somehow achieved in water the dispersibility is still poor. Using the present method, on the other hand, a dispersion of fine noble metal powder offering very high purity and comprising particles of uniform shape and size can be produced efficiently. The obtained fine powder (fine particles) should be called "super-fine particles," because their particle size is far smaller than that of normal fine powder.

One feature of the present invention lies in its utilization of super-fine noble metal particles of micron to nano-order, which are much smaller than fine noble metal particles. Super-fine noble metal particles offer markedly better dispersibility and health functions compared with fine particles, and in the context of the present invention "micro-dispersion water/solution" refers to a solution in which the aforementioned newly developed "super-fine particles" are dispersed.

The mechanisms as to why the noble metal materials obtained by the present invention provide an effective head massaging solution, as well as the details of bioactivity embodied by such materials; are not yet clear. Studies are being conducted to uncover these mechanisms and bioactivity.

In order to achieve practical applications of the health-promoting function and disease-curing efficacy of these noble metal materials and develop them into effective new technologies, the inventors conceptualized that by utilizing a method for producing dispersion water of super-fine metal particles filed earlier by the inventors (Japanese Patent Application No. 2001-315205) and applying it to produce a solution in which super-fine noble metal particles are dispersed, these functions of noble metals could be effectively embodied. The inventors also found that use of the obtained solution embodying the above functions in clothes or head treatment products would sufficiently answer the consumer's needs for health promotion and cleanliness mentioned earlier. These findings have led to a successful development of the present invention.

The present invention mainly comprises micro-dispersion water in which super-fine particles of noble metals such as gold, silver and platinum are dispersed. This micro-dispersion water is produced by the below-mentioned method for producing dispersion solution of super-fine noble metal particles as developed by the inventors.

Of noble metals, gold and platinum particles do not dissolve or disperse in water. Therefore, even when a dispersed state of fine gold or platinum particles is somehow achieved in water, the surface area of particles is small relative to the weight of gold or platinum mixed into water, which results in poor cost-efficiency. The dispersed particles will also settle quickly. Therefore, any method that achieves such temporary dispersion does not satisfy the technical requirements.

Fig. 1 outlines the method for producing micro-dispersion solution of super-fine noble metal particles as proposed by the present invention.

The basic production method proposed by the present invention is to continuously or intermittently feed water into a tank and cause plasma discharge in water between an electrode made of noble metal and its counter electrode in order to heat the noble metal electrode to high temperatures and thereby generate noble metal vapor, and then cause the generated noble metal ion vapor to instantly contact, and micro-disperse in, water. At this time, very small super-fine noble metal particles of micron to nano-scale are produced and micro-dispersed.

In this process, vibrating or sliding the noble metal electrode and its counter electrode on the high-voltage discharge generator will prevent the noble metal electrode from fusing, and the amount of micro-dispersion can be controlled easily due to achievement of instant arcing. Since the noble metal electrode will be consumed as super-fine particles, an electrode having a bar or wire shape is fed in accordance with the rate of consumption. Additionally, there is no need to select a different power supply for a given purpose, because the amount of current flowing through the circuit can be adjusted easily by changing the diameter and length of the counter electrode made of carbon, etc.

The produced water is retrieved from the outlet using the discharge pump and can be used directly without filtering. Depending on the application, filtering the water using a filter system, etc., will remove a trace amount of fine noble metal particles with a larger particle size and help yield micro-dispersion water in which only super-fine particles are micro-dispersed. The residue of fine noble metal particles left on the filter can be recovered by backwash and reused, as necessary, to improve economy.

A mixture gas of hydrogen and oxygen provides the most efficient and stable means for burning noble metal in water, and high pressure is needed to achieve stable combustion.

Study is underway to find a physicochemical explanation as to why noble metal materials melt instantly in a combustion gas atmosphere under high-pressure water and become super-fine particles.

Thus obtained noble metal dispersion water can be used as a material for the head massaging solution proposed by the present invention. The produced super-fine noble metal particles have very strong hydrophobicity and therefore achieve a stable dispersion state. These super-fine noble metal particles will remain dispersed in a stable manner for a long period without agglutinating, settling or otherwise separating from water.

It is presumed that partly due to their ion-releasing effect, very large active surface area and micro-interaction with water molecules, and partly due to the unique characteristics of the noble metals themselves, these super-fine noble metal particles will, if applied to the head and followed by head massage, directly contact the skin to activate the body cells or promote muscle metabolism, hemocatharsis, etc., through the physiological activation points in the key areas of the body.

### "Example"

An example of the present invention is explained according to the drawings. Note, however, that the present invention is not limited to this example.

### Production of Head Massaging Solution

The noble metal micro-dispersion water as proposed by the present invention is produced using the apparatus shown in Fig. 2.

Shown in Fig. 2 is an apparatus for producing micro-dispersion water in which super-fine noble metal particles are micro-dispersed, comprising a power supply for high-voltage/current discharge (2), a high-voltage discharge generator (1) equipped with a noble metal electrode (6) and its counter electrode (7), a water tank (3), a water inlet to the water tank (8), an outlet for produced micro-dispersion water of super-fine noble metal particles (9), and a discharge pump (10). An electrode feeder (4) for feeding the electrode pair on the high-voltage discharge generator, an electrode-vibrating/sliding device (5) for vibrating or sliding the electrodes and a filter system (11) for filtering the obtained micro-dispersion water are attached as adjuncts.

The production apparatus proposed by the present invention comprises the water tank (3) made of metal, or preferably steel, wherein water is supplied from the inlet (8) and the metal electrode (6) and its counter electrode (7) on the high-voltage discharge generator (1) are submerged in water. By charging current between the electrodes in this condition, plasma discharge will occur and noble metal ions will instantly separate from the noble metal electrode and micro-disperse in water, thereby producing water in which super-fine noble metal particles are micro-dispersed.

When causing plasma discharge between the noble metal electrode and its counter electrode, vibrating or sliding both electrodes will prevent electrode fusion, and instant arcing will enable an easy control of micro-dispersion amount.

The obtained micro-dispersion water can be used directly, or fed by the discharge pump (10) to pass through the filter system (11) to obtain filtered product (12). A desirable filter system is one using hollow-fiber membranes of micron order mesh, instead of ionexchange membranes or reverse-osmosis membranes, in order to filter only super-fine particles through.

Under the present invention, achievement of pulsed plasma discharge will allow for a near-continuous production of micro-dispersion water of super-fine noble metal particles, even when the production volume is increased. Since the apparatus can also be constructed in a small size, the present invention provides an efficient production method. The simple apparatus is also safe, because it does not require overly high pressure resistance.

If fine noble metal particles of relatively large size must be removed from the produced micro-dispersion water, it is desirable to attach a filter system (11) to this apparatus as an adjunct.

To refine the produced water, pass it through a filter system (11) comprising hollow-fiber membranes of micron order mesh, as explained above, to remove the produced fine noble metal particles as necessary.

Filtering will provide drinking water meeting the food sanitation standards or cosmetic lotion meeting the cosmetics material standards. One example of filtration using hollow-fiber membrane filter is to provide hollow-fiber membranes of 50 microns; 25 microns, 3 microns, 0.5 micron and 0.1 micron at the point where the produced water is discharged from the reaction tank, and then let the water pass through these membranes sequentially. Filtered fine noble metal particles can be recovered via backwash and reused for added economy.

A gold or platinum bar is used as the noble metal electrode, while the same metal or carbon is used as the counter electrode, and the two electrodes are charged with electricity for one hour. The electrodes may be fixed plates, or bars or wires that are successively fed using an auxiliary device. Either way, the electrodes are vibrated or slid by the electrode-vibrating/sliding device (5), and plasma discharge is caused in water between the noble metal electrode and its counter electrode by supplying power to the high-voltage discharge generator (1).

### Structure of the Head and Action of Micro-Dispersion Water of Super-Fine Noble Metal Particles

Our head comprises the skull, in which brains that implement an integrated control of the various functions of our body are encased. Emotions are also considered a product of brain control. The cerebrum occupies 80% of the total brain mass and plays an important role of collecting information from, and sending commands to, each part of the body. On the other hand, the cerebellum adjusts the motor instructions issued by the cerebrum and sends them to each part of the body. The brainstem is where the nerve fibers concentrate. The nerves system controls the functions of cells, tissues and organs and transmits information needed to cause our body to adapt to changes. occurring inside and outside the body.

The noble metal contained in a micro-dispersion solution of super-fine noble metal particles offers excellent electrical stability, among other physical properties, and thereby facilitates the aforementioned transmissions of electrical signals through the nerves and consequently improves the functions of our brains and nerves in controlling the various parts of our body.

The specific effects that are expected from such micro-dispersion solution include correction of autonomic nerves functions, stabilization of hormonal balance and improvement of motor and sensory functions.

### How to Massage the Scalp

Applying a micro-dispersion solution of super-fine noble metal particles to the scalp alone does not yield significant brain activation effect. In fact, the brain activation effect can be doubled by a subsequent massaging of the head. Various methods for massaging the head are known, such as the method to promote hair growth by massaging the scalp using various equipment and thereby facilitating the permeation of a medical solution (Japanese Patent Application Laid-open No. 1-146551), and the method that comprises the steps to moisten the scalp with active water containing 16 ppm of dissolved oxygen, irradiate visible light and laser light, apply a hair tonic, and then massage the head using air pressure (Japanese Patent Application Laid-open No. 7-16304). After diligent studies, however, the inventors found a new way to activate the brains by way of applying water containing micro-dispersed super-fine noble metal particles to the scalp and then massaging the head.

Steps (A) through (C) below explain an example of the aforementioned massaging method after application of a micro-dispersion solution of super-fine noble metal particles:
(A) Place the palms of the hands over the head to warm the scalp, in order to let the scalp absorb the micro-dispersion solution of super-fine noble metal particles.
(B) Move the fingers by burying them into the hairs and then lifting up to loosen the scalp. Work form the top of the head toward the sides, where the aforementioned finger movements should cause the tails of the eyes to rise.
(C) Confirm that the hard scalp has loosened and softened.

By applying a newly developed micro-dispersion solution of super-fine noble metal particles to the scalp and massaging the head, as proposed by the present invention, the solution permeates through the hair roots into the hair tissues in the scalp all the way to the hair matrix cells and hair papilla, thereby transferring the beneficial effects of the solution to the tissue nerves and capillary vessels to produce remarkable effects in the activation of the brains, as confirmed by tests.

### Effectiveness Verification of Micro-Dispersion Solution of Super-Fine Noble Metal Particles

The specific steps of scalp massage as proposed by the present invention involve cleaning the scalp by shampooing and drying the wet hair using a towel or dryer.

In a specific experiment, the head was placed in the shampoo basin and the hair was washed with hands by rubbing actions for five minutes using a VIDAL SASSOON brand shampoo (made by Max Factor). Thereafter, the shampoo was rinsed off thoroughly using warm water. To dry the wet hair, a dryer was used to apply hot air for three minutes and cold air for 10 minutes. Next, with the face turned upward a micro-dispersion solution in which super-fine noble metal particles are micro-dispersed was carefully applied to the scalp using a sponge by making sure the solution did not drip. Thereafter, the head was massaged for 10 minutes using the acupressure technique.

To examine the effect of applying to the scalp a micro-dispersion solution in which super-fine noble metal particles are micro-dispersed and then massaging the head, an example using gold as the noble metal was examined.

### Massaging Techniques

Various massaging techniques may be used with the present invention, such as the pressuring technique, acupressure technique, tapotement technique, stroking technique and rubbing technique. These techniques produce significant effects if applied on the activation points along the autonomic nerves and other nerves. Any of these massaging techniques may be used after applying a micro-dispersion solution in which super-fine noble metal particles are micro-dispersed, as long as they can relieve stress and anxiety and stabilize the autonomic nerves functions, etc.

### Effects on Lateral Dynamic Visual Acuity of Spraying Micro-Dispersion Solution of Super-Fine Gold Particles over the Head

Lateral dynamic visual acuity (DVA) is the ability required to see an object moving in the lateral (horizontal) direction and therefore provides a measure of one's essential and most important visual function in daily life or while driving a car-the visual function of accurately recognizing a moving object. Here, the effects a micro-dispersion solution containing gold may have on lateral dynamic visual acuity were verified.

### Effectiveness Verification Test on Lateral Dynamic Visual Acuity

○ Subjects: 10 females aged 19 to 25 years old
○ The details. of the experiment and content of drinking water were not explained prior to the test. However, the measuring method was explained in detail and five practice sessions were conducted. The initial DVA of each subject was in the standard range expected of her age group.
○ Measuring. apparatus and its setup: A lateral dynamic visual acuity tester-10, wherein a horizontally moving Landolt ring corresponding to a visual acuity of 0.025 is shown on a circular-arc screen installed 90 cm forward of the subject's eyes, was used. The 'initial speed of ring movement was set to 40 rpm, and the speed was gradually decreased.
○ Judgment condition: The subject's face was fixed on the chin support, and the moment the subject recognized the direction of the cut in the Landolt ring the switch was pressed and the corresponding ring-movement speed was displayed digitally. The ring-movement speed when the cut in the presented Landolt ring was correctly recognized was used as the test result, and the measurements taken by five tests were used as the subject's DVA.
○ Spray condition of micro-dispersion solution of super-fine gold particles to the head: The subjects sprayed 5 mL of the micro-dispersion solution of super-fine gold particles twice a day, once in the morning and once in the evening.

The solution was used for 30 days, and DVA was measured after every 10 days.

The control group used normal tap water as a placebo.
○ Results: Table 1 shows the DVA values of subjects using the micro-dispersion solution of super-fine gold particles and those using placebo water, measured after different numbers of days:

**Table 1**

| Elapsed days | Use of micro-dispersion solution of super-fine gold particles | Use of placebo water |
|---|---|---|
| Initial | 34.0 | 33.4 |
| 10 days | 34.6 | 33.6 |
| 20 days | 35.0 | 34.4 |
| 30 days | 35.4 | 34.4 |

As shown by the results in Table 1, the DVA of subjects using the micro-dispersion solution of super-fine gold particles shows a significant difference of around 5% compared with the initial value. It was therefore found that DVA would be improved by spraying the micro-dispersion solution of super-fine gold particles over the scalp twice a day for 30 days. In other words, it is presumed that the micro-dispersion solution of super-fine gold particles has some kind of positive effects on our visual function as well as the information processing capability of the brain.

### Effects on Brain Fatigue of Spraying Micro-Dispersion Solution of Super-Fine Gold Particles over the Head

Brain fatigue is understood as a cause of broad weakening of brain function, affecting the areas of the brains that control mental activities such as thinking. The level of brain fatigue was measured using a brain fatigue meter.
○ Subjects: 12 healthy adult males aged 20 to 26 years old
○ Preliminary examination: The subjects underwent preliminary examination over a period of 20 days prior to the test to obtain their normal brain fatigue level. The test was conducted as a blind test, in which the subjects were not told of the details of the micro-dispersion solution of super-fine gold particles or whether they were given a placebo.
○ Measuring method and judgment: The subject was shown a measuring apparatus placed 50 cm forward of his eyes. On the apparatus numbers 0 to 9 were displayed electronically with one of them placed in a different order and that number was caused to flash at frequencies of 1 to 10 Hz increased by 0.5 Hz at a time. The subject was instructed to determine the flashing number within the time limit.

When the subject gave the correct answer, the flashing frequency was increased by 1 Hz and the same process was repeated until the subject exhibited difficulty choosing the answer. The Hz value in this condition was used as the brain fatigue level.
○ Spray condition of micro-dispersion solution of super-fine gold particles: The subjects were instructed to spray the solution twice a day, once in the morning and once in the evening, continuously for 30 days, and their brain fatigue level was measured after every 10 days.
○ Results and judgment: Table 2 shows the measured brain fatigue levels.

**Table 2**

| Elapsed days | Spraying of micro-dispersion solution of super-fine gold particles | Spraying of placebo water |
|---|---|---|
| Initial | 6.7 | 6.7 |
| 10 days | 7.2 | 7.1 |
| 20 days | 7.4 | 7.3 |
| 3 0 days | 8.1 | 7.4 |

As shown by the results above, no significant difference was noted after 20 days. However, the results of both groups exhibited a significant difference of around 5% after 30 days. This confirms that the micro-dispersion solution of super-fine gold particles would suppress brain fatigue if used continuously for 30 days.

### Effects on Brain Waves of Spraying Micro-Dispersion Solution of Super-Fine Gold Particles over the Head

Various autogenic training methods are used of late to relieve physical and psychological stress. However, data suggest that such training implemented for the purpose of relieving stress can actually become a strong mental stressor. For this reason, mental control has been cited as an important element in the recent autogenic training methods. However, many of the specific mechanisms as to which brain function is affected in which way by autogenic training, being implemented as a means for mental management, remain unknown. Here, the theta brain wave, which is currently known as the most accurate indicator of the condition of brain function, was used as a guide to examine how theta wave presentation would be affected by spraying a micro-dispersion solution of super-fine gold particles over the head. The expectation was that if the solution showed positive effect on theta wave presentation, it would mean spraying the solution over the head could provide an effective means for rational mental management.
○ Brain wave test method: The brain wave test used Fmθ wave as a marker. Fmθ wave presents along the medio-frontal line of the head during mental work and has a wavelength of 4 to 7 c/sec. It continues for one second or longer and the amplitude is known to be 18 ± 1.2 µV for males and 22 ± 1.7 µV for females.

This brain wave is associated with specific sensations, and generally the wave presents in 20% of the population. Of these, only around 20% experience sustained presentation of Fmθ wave.
○ Subjects: 7 adult females aged 19 to 25 years old
Based on an informed consent the subjects underwent preliminary examination to set the initial level.

The test was conducted as a double-blind test, in which the subjects were not told of the purpose or content of the test.
○ Condition of experiment: The subjects were given a micro-dispersion solution of super-fine gold particles or placebo comprising general tap water and instructed to spray them over their head.

The subjects sprayed their respective test water twice a day, once in the morning and once in the evening, continuously for 30 days. Examination was conducted after every 10 days.
○ Procedure of experiment: First, the presentation ratio of Fmθ was calculated by measuring the normal brain waves, and the obtained value was used as the baseline. Thereafter, psychological test, measurement of brain waves with the eyes open in resting state, and one minute and ten minutes of Kraepelin continuous addition task were conducted, after which the subjects rested for 10 minutes and then again underwent psychological test.

The examination results and evaluation are shown below.

The values in the table indicate the Fmθ numbers measured after different numbers of days.

**Table 3**

| Elapsed days | Spraying of micro-dispersion solution of super-fine gold particles | Spraying of placebo water |
|---|---|---|
| Initial | 29.5 | 29.5 |
| 10 days | 31.0 | 28.0 |
| 20 days | 37.0 | 34.0 |
| 30 days | 42.0 | 32.0 |

As shown by the results above, a significant difference of around 1% was noted after 30 days. Therefore, it is suggested the micro-dispersion solution of super-fine gold particles, if used continuously for 30 days, would cause a significant increase in the presentation of Fmθ wave and thereby effectively stimulate the cerebral nerves functions and mental activities, particularly concentration.

(The details of the above utility test of head massage are explained in the report submitted on October 30, 2001.)

### Industrial Field of Application

It was found that when a solution, which contains super-fine noble metal particles as obtained by causing plasma discharge in water between a noble metal electrode and its counter electrode, is applied to the scalp and then the head is massaged, as proposed by the present invention, such solution would permeate through the hair roots into the hair tissues in the scalp all the way to the hair matrix cells and hair papilla, thereby transferring the beneficial effects of such solution to the tissue nerves and capillary vessels to produce remarkable effects in the activation of the brains.

## Claims

1. A head massaging solution comprising a micro-dispersion solution of super-fine noble metal particles, wherein said micro-dispersion solution is produced by causing plasma discharge in water between a noble metal electrode and its counter electrode.

2. A method for. treating a head comprising:
applying micro-dispersion water containing super-fine noble metal particles to a scalp; and
massaging a head.
